# EUROPEAN PATENT SPECIFICATION

(11) **EP 4 116 352 B1**
(45) Date of publication and mention of the grant of the patent: **27.08.2025**
(21) Application number: 22182831.2
(22) Date of filing: 04.07.2022
(51) Int. Cl.: C08G 63/06, C08G 63/89, C07C 51/09, C11B 13/04, B32B 27/36

(54) **PROCESS FOR THE EXTRACTION OF CUTIN FROM TOMATO PROCESSING WASTE**
VERFAHREN ZUR EXTRAKTION VON CUTIN AUS RÜCKSTÄNDEN DER TOMATENVERARBEITUNG
PROCEDE D'EXTRACTION DE CUTIN A PARTIR DE DÉCHETS DE TRANSFORMATION DE TOMATES

(30) Priority: 07.07.2021 IT 202100017945
(43) Date of publication of application: 11.01.2023
(73) Proprietor: Tomapaint S.r.l., 43121 Parma (PR) (IT)
(72) Inventor: Montanari, Angela, 43121 Parma PR (IT); Barbieri, Tommaso, 43121 Parma PR (IT); Chiesa, Stefano, 43121 Parma PR (IT)
(74) Representative: Perani & Partners S.p.A.

(56) References cited:
- WO-A1-2015/028299
- WO-A1-2020/260312

## Description

### FIELD OF THE INVENTION

The technical field to which the invention belongs is that of the extraction methods of a natural polyester, called cutin, from tomato processing waste.

### STATE OF THE ART

Cutin is a natural cross-linked polyester, mainly formed by condensed polyhydroxy acids. Cutin is located in the cuticle of the plants, of which it is the main component (40-85%, w/w). The cuticle is a hydrophobic layer deposited exclusively on the outside of the epidermis cells. The primary role attributed to the plant cuticles is to avoid the water loss from internal tissues; moreover, the cuticle also has a function of gas barrier and thermal regulation, as a defence against pathogens, reduction of nutrients leaching and protection from mechanical injury and UV radiation damage.

The by-products of tomato processing are a rich source of cutin and their recovery is considered important from an economic and environmental point of view. The tomatoes world production is about 170 million tons per year, mainly concentrated in the northern hemisphere, of which about 38 million tons are processed into different products, such as tomato purée, diced tomato, tomato paste, peeled tomatoes and tomato skins. The waste produced, peels and seeds, every year, at an industrial level, are significant: about 1.0 million tons, of which 300,000 tonnes in Europe alone.

Chemically, cutin is defined as a polymeric network of C16 and C18 polyhydroxylatedfatty acids cross-linked by ester bonds (A. Heredia, 'Biophysical and biochemical characteristics of cutin, a plant barrier biopolymer', Biochimica et Biophysica Acta 1620 (2003) 1-7). Many of them are exclusively C16 fatty acids belonging to the family in which 10,16-dihydroxyhexadecanoic acid, and its positional isomer 9,16-dihydroxyhexadecanoic acid, are the main components of cutin. Only a small fraction of cutin is made up of fatty acids belonging to the C18 family, the most abundant components of which are 9,10-epoxy-18-hydroxyoctadecanoic and 9,10,18-trihydroxyoctadecanoic acids, although some derivatives may be present in the form of unsaturated cutin (G. M. Lopez "Biomechanics of the epidermis and skin of the fruit of tomato (solanum lycopersicum l.) and its relationship with the cracked" (2006), Ph. Thesis, Faculty of Sciences, Department of Molecular Biology and Biochemistry, University of Malaga).

Cutin is generally extracted from plant material following isolation of the cuticle. Enzymes such as pectinases and cellulases in acetate buffer are usually used for cuticle isolation. Isolated plants cuticles are dewaxed by immersion in organic solvents, and polysaccharides are removed by acid hydrolysis. However, the degradation efficiency of polyesters by cutinases is too low for industrial applications.

In a recent paper, a structure-based protein engineering strategy was successfully used to improve the hydrolysis capacity of a *T*. *fusca* cutinase (Q.Dong, S. Yuan., L. Su, Q. Zhao, J. Wu, W. Huang and J. Zhou 'Structure-guided engineering of a Thermobifida fusca cutinase for enhanced hydrolysis of natural polyester substrate', Bioresources and Bioprocessing, 2020, 7:37)

In the enzymatic method, the cuticular membrane is isolated by means of enzymatic treatment to degrade the polysaccharides. The cuticle is then extracted (solid-liquid extraction using a Soxhlet extractor with the solvent CHCl₃ and MeOH) to remove soluble material, waxes and other small molecules, obtaining cutin-enriched residues (R. Jarvinen, A. J.D. Silvestre, A. M. Gil, H. Kallio, "Solid state 13CP-MAS NMR and FT-IR spectroscopic analysis of cuticolar fractions of berries and suberized membranes of potato,' Journal of Food Composition and Analysis 24 (2011) 334-345; H. Kallio, R. Nieminen, S. Tuomasjukka, M. Hakala 'Cutin composition of five Finnish berries' Journal of Agricultural and Food Chemistry 54 (2006), 457-462).

Only dewaxed and sugar-free peels can be obtained with this method, but not cutin. This method lacks a procedure to isolate cutin from the dewaxed peels. Furthermore, this method is expensive in terms of solvent use; it should also be considered that Soxhlet extraction uses toxic organic solvents, such as chloroform (carcinogenic) and methanol, and this extraction must be repeated to increase extraction yields. Thus, the procedure is not particularly environmentally friendly.

Cutin can be depolymerized by cleavage of the ester bonds by means of alkaline hydrolysis, using NaOH or KOH in water, transesterification in methanol containing BF₃ or NaOCH₃, reductive cleavage by treatment with LiAlH₄ in THF, or by TMSI in organic solvents. These methodologies are unsuitable for large-scale cutin extraction due to the many steps involved and the high costs of solvents and reagents.

Another solvent used to extract cutin is acetone, as reported in the US patent application No. US 2011/0319504, in which cutin is isolated by extraction with acetone in a Soxhlet apparatus and then with a reflux system in a basic solution. This method was tested; however, the final extract did not have the usual cutin appearance. As proof of this, the IR spectrum carried out on the product did not show the usual appearance of cutin spectrum. Furthermore, this method is expensive both in terms of solvent used and time.

Finally, another possibility reported in the literature to extract cutin is by acid hydrolysis (J. J. Benitez, R. Garcia-Segura, A. Heredia, 'Plant biopolyester cutin: a tough way to its chemical synthesis', Biochimica et Biophysica Acta 1674 (2004) 1-20 3). In this method, cutin samples were obtained following hydrolysis of the dewaxed cuticles in a 6 M HCl solution for 12 h at 105°C to remove the hydrolyzable polar components and subsequently depolymerized in a 3% (w/v) sodium methoxide solution for 18 h at 100°C. After the extraction of the tomato cutin monomers in an organic phase (diethyl ether), the solvent was evaporated in order to quantify and identify the cutin monomers by gas chromatography-mass spectrometry. This method was tested, but the yield was very low, and the extract showed little ability to form a new bio-lacquer.

The international patent application WO 2017/100636 describes an extraction process that provides a cutin with a high degree of purity; however, the process, which uses organic solvents and high temperatures and pressures, is particularly complex and expensive.

The international patent application WO 2020/260312 describes a method for preparing a polymer of the elastomer type based on a monomer selected from polyhydroxylated fatty acids and the esters of a polyhydroxylated fatty acid and a C1-C18 aliphatic chain alcohol. The method comprises preparing a reaction medium by mixing the monomer with a polyol, this reaction medium being catalyst free, then a step of heating the reaction medium in order to implement the copolymerisation of the monomer and of the polyol and the cross-linking of the polymer thus formed, with at least the initial phase of this heating state being implemented at a reduced pressure.

### Problem of the state of the art

As highlighted above, the methods known to state of the art are mainly based on extraction processes with organic solvents, which are known to make more hazardous and expensive the treatment of the waste.

The enzymatic processes, although suitable for the purpose, have a degradation efficiency that is too low, which makes them not economically advantageous and of low interest for industrial applications.

In cases where the acid hydrolysis was used, followed by depolymerization and extraction in the organic phase, the product obtained was found to have a low yield and a poor ability to form effective bio-lacquers/bio-resins.

The European Patent No. EP3039064 (B1) describes a method for extracting cutin from tomato peels that does not use organic solvents. The method, in particular, includes the steps of
- dipping the peels in an alkaline solution and heating;
- filtering the alkaline suspension containing the tomato peels to remove the solid residue and keep the liquid residue;
- acidifying the liquid residue by adding HCl until a pH of 5 or 6 is reached;
- centrifuging the suspension obtained from acidification to discard the liquid residue and retain the solid residue.

The solid residue obtained is a cutin extract characterised by:
- a water content between 25 and 80% (v/v);
- a 10, 16-dihydroxyhexadecanoic acid yield between 60 and 80% (w/w).

The method under patent EP3039064 uses only tomato peels, which sometimes contain contaminants resulting from farming or tomato processing.

In addition, the use of HCl makes the extraction method known to the state of the art unsustainable due to the chloride salts formed during the acidification phase: for this reason, the liquid residue cannot be discharged into surface waters or biogas plants.

### SUMMARY OF THE INVENTION

In this context, the technical task at the basis of the present invention is to provide an improved cutin extraction process compared to the one described in the patent EP3039064.

The first object of the present invention is, therefore, a process for the extraction of cutin from tomato waste including the steps of:
A. Preparing waste or by-products obtained from tomato processing;
B. Soaking the tomato waste in an alkaline solution to obtain an alkaline mixture having a pH > 10, the alkaline mixture comprising a liquid phase and a dispersed solid residue;
C. Subjecting the alkaline mixture to heat treatment, at a temperature > 100°C and ≤ 130°C, for a period of time ≥ 10 minutes and < 4 hours;
D. Isolating the liquid phase of the heat-treated alkaline mixture from the solid residue, the liquid phase being an alkaline cutin solution, the solid residue being an exhausted tomato peel waste free of cutin;
E. Acidifying the alkaline cutin solution by adding an organic acid, so as to obtain an acidified cutin suspension with a pH < 5, the acidified cutin suspension comprising a liquid phase and a precipitate;
F. Isolating the precipitate of the acidified suspension of cutin from the liquid phase by means of centrifugation using a horizontal decanter and wash the precipitate with water,
the precipitate obtained being a cutin extract having a 10, 16-dihydroxyhexadecanoic acid purity between 50% and 80% by weight of the total weight of the extract (w/w). Further objects of the present invention are:
- an exhausted tomato peel waste free of cutin as defined in claim 8 and its uses;
   - a bio-resin comprising the cutin extract obtained by the process according to the present invention and its uses;
   - a multilayer article comprising: a first layer, comprising or consisting of paper, plastic, bio-plastic, metals, metal alloys, wood; a second layer adjacent to the first layer, comprising or consisting of the bio-resin according to the present invention.

### Advantages of the invention

The process of the invention allows the extraction of cutin from tomato processing waste and its reuse in further formulations.

In particular, the process according to the invention provides for the use, in addition to the tomato peels, of generic waste or by-products from the processing of fresh tomatoes from preserved industries. The use of waste or by-products from the processing of tomatoes from organic cultivation also reduces the risk of the presence of contaminants in the peels.

The process of the invention eliminates the use of HCl, replacing it with organic acids. The use of organic acids makes it possible to reduce the risks of toxicity during the processing steps; to reduce the risks of environmental contamination; to reduce the contamination by sodium chloride of the wastewaters used during the process. Sodium chloride is difficult to eliminate, and it is subject to strict environmental legislation.

In this sense, the described cutin extraction process allows not only the valorisation of these tomato processing waste, but also the recycling of the reaction products.

Compared to the process described in EP3039064, the method according to the present invention allows to reduce the processing time, having the Applicants identified suitable temperatures and heating times to speed up the depolymerization phase in alkaline solution without causing degradation of the cutin contained therein.

Still advantageously, the described cutin extraction process allows the production of a bio-resin, starting from tomato processing by-products, with potential application in the preparation of lacquers, coatings, water-based paints, and cosmetic formulations.

Also advantageously, the process for the extraction of cutin allows the use of this bio-resin for the production of multilayer articles preferably for wrapping or packaging, with a consequent reduction in the purchase costs of the raw materials for the production of such articles.

Finally, the process makes it possible to obtain exhausted tomato peel waste free of cutin, which finds application as a raw material for producing food supplements or additives, or as a substrate for extracting carotenoids/lycopene.

### DETAILED DESCRIPTION OF THE INVENTION

### Extraction process

As mentioned above, the object of the present invention is a process for the extraction of cutin.

### A. Preparing waste or by-products obtained from tomato processing.

In a step A of the extraction process, waste or by-products obtained from tomato processing are prepared.

Preferably, by tomato waste or by-products is meant the material from fresh tomato processing, selected from the group consisting of peels, seeds, leaf by-products, green tomatoes, peel residues and mixtures of the previous. Preferably, tomato waste or by-products are peels or combinations of peels and seeds.

In accordance with a preferred embodiment, the waste or by-products of tomatoes are tomato peels. It should be noted that different types of peels can be used: peels coming directly from tomato processing, dried peels, frozen peels and peels from which lycopene has been extracted.

According to a preferred embodiment, the tomato waste or by-products preparation step (A) comprises the following sub-steps:
*A1.* separating the peels from the tomato waste or by-products, preferably by flotation with running water;
A2. squeezing the separated peels to reduce their water content; it should be noted that, preferably, the peels that have not yet been squeezed have a solid content of about 20%.

According to a preferred embodiment, the seeds separated from the peels are intended for the biogas production, animal feed or used for the extraction of oil and proteins.

Preferably, the water used for flotation is recirculated through a vertical centrifuge to remove residual tomato pulp and to clean the water; in this way, the water can be used again for the same purposes, thus minimizing its consumption.

The pulp removed by centrifugation is preferably used for the biogas production.

### B. Soaking tomato waste in an alkaline solution

The tomato waste obtained from the preparation step (A) are immersed in an alkaline solution to obtain an alkaline mixture with a pH > 10. Thealkaline mixture comprises a liquid phase and a dispersed solid residue.

Preferably the alkaline mixture has a pH ≥ 10.5 and ≤ 14, preferably ≥ 11 and ≤ 13.5, preferably equal to about 13.

According to a preferred embodiment, the alkaline solution is a solution comprising one or more basic substance chosen from the group consisting of: NaOH, KOH.

Preferably, the basic substance is NaOH.

According to a preferred embodiment, the alkaline solution has a concentration between 0.5 and 6 M, preferably between 0.5 M and 3 M, preferably between 0.5 M and 1.5 M, preferably between 0.5 M and 0.9 M, preferably equal to about 0.75 M.

According to a preferred embodiment, the combination ratio (weight/volume) of the tomato waste or by-products (weight) with the alkaline solution is between 0.1 and 10.

### C. Heat treatment of the alkaline mixture at a temperature > 100°C and ≤ 130°C, for a period between ≥ 10 minutes and < 4 hours.

The alkaline mixture obtained from the immersion step (B) is heat treated at a temperature preferably between 105°C and 130°C, preferably between 110°C and 130°C, preferably between 115°C and 130°C, preferably equal to about 125°C.

According to a preferred embodiment, the heat treatment is carried out for a period of time preferably between 20 minutes and 2 hours, preferably between 20 minutes and 1.5 hours, preferably between 20 min and 60 min, preferably between 20 minutes and 45 min, preferably equal to about 20 minutes.

### D. Isolation of the liquid phase of the thermal treated alkaline mixture from the solid residue.

The term "isolation" refers to the separation of one or more phases from a suspension. Suitable techniques, for the purposes of the invention, to carry out the isolation step are those known to the person skilled in the art, preferably chosen between filtration and centrifugation.

Preferably, centrifugation is performed by means of a horizontal centrifuge, decanter.

The liquid phase is an alkaline solution of cutin. Note that the liquid phase has a brown or yellow colour and does not contain fine solid particles.

The solid residue is exhausted tomato peel waste, free of cutin.

Preferably, the exhausted tomato peel waste is characterised by a solid content of between 20% and 30% (w/w).

For the purposes of the present invention, the term exhausted tomato peel waste is understood to mean a tomato peel waste which is no longer suitable for re-entry in the preparation of tomato peel waste step (A) according to the process of the present invention. Cutin-free peel waste means a peel waste having, when dried, a cutin content < 30%, preferably between 30% and 20% by weight on the total weight of the exhausted peel waste.

It should be noted that, preferably, the peel waste, when wet, is characterized by a cutin content between 6 and 12% (w/w).

### E. Acidification of the alkaline cutin solution by addition of an organic acid and obtaining of an acidified cutin suspension with a pH ≥ 2 and < 5.

The liquid phase obtained from the isolation step (D) consists of an alkaline solution of cutin.

Acidification is preferably carried out until a pH preferably comprised between 2 and 5, preferably between 3 and 5, preferably between 4.3 and 4.5.

The acidified cutin suspension comprises a liquid phase and a precipitate.

Preferably, the organic acid is chosen from the group consisting of: acetic acid, citric acid, formic acid, oxalic acid, succinic acid, glutaric acid, maleic acid.

Preferably, the organic acid is citric acid or acetic acid.

More preferably, the organic acid is added in the form of an aqueous solution, said aqueous solution preferably having a concentration between 1M to 3M, preferably equal to about 2.5M.

The organic acid is preferably added to the alkaline solution at a concentration between 10% and 20% by volume on the total volume of the solution (v/v), preferably 13%.

The organic acid is preferably added to the alkaline solution until a colour change of the solution from brown to ochre is observed.

All the acids considered give an IR spectrum of cutin in which the main peak is around 1700-1705 cm⁻¹ and there is an area of the spectrum for values below 1680 cm⁻¹ sufficiently clean.

### F. Isolation of the precipitate of the acidified cutin suspension from the liquid phase and washing of the precipitate with water.

The isolation step (F) yields a cutin extract characterised by 10,16-dihydroxyhexaenoic acid purity between 50% and 80%, preferably about 60% by weight on the total weight of the extract (w/w).

Preferably, the cutin extract obtained from the isolation step (F) has a water content between 25% and 40% by volume on the total volume of the extract (v/v).

Preferably, the cutin extract obtained from the isolation step (F) has a solid content between 60% and 80% by weight, on the total weight of the extract (w/w).

More preferably, the cutin extract obtained from the isolation step (F) has a sugar content between about 0.01% and 0.2% by weight on the total weight of the extract, preferably between about 0.05% and 0.2% (w/w), preferably between about 0.05% and 0.1% (w/w).

According to a preferred embodiment, the cutin extract obtained from the isolation step (F) has a fat content between about 1.0% and 5.0%, preferably between about 2.0% and 4.0% by weight on the total weight of the extract (w/w).

Still preferably, the cutin extract obtained from the isolation step (F) has a lycopene content between about 0.04% and 0.06%, preferably equal to about 0.05% by weight on the total weight of the extract (w/w).

It should be noted that the extraction process, according to the invention, allows the cutin extract to be obtained with extraction yields, relative to the initial by-products, comprised between 10% and 30%, preferably between 10% and 20%, preferably between 10% and 15%, preferably equal to about 13%.

Preferably, the isolation step (F) is performed by horizontal decanter centrifugation, preferably at a rotational speed between 4500-5500 rpm, preferably at 5000 rpm.

The use of the horizontal decanter centrifuge advantageously allows to conduct the isolation phase (F) continuously, preferably obtaining an extract quantity of about 150 kg/hour, corresponding to about 1 kg/hour, corresponding to about 100 g/min.

In accordance with a preferred embodiment, the liquid phase obtained from the isolation step (F) is re-entered into the immersion step (B) as an alkaline suspension of cutin, after alkalinization to a pH > 10, preferably pH ≥ 10.5 and ≤ 14, preferably ≥ 11 and ≤ 13.5, preferably equal to about 13.

Alternatively, the liquid phase obtained from the isolation step (F) is discarded.

According to a preferred embodiment, the step of washing with water provides that the precipitate (volume) and water (volume) are combined in a volume/volume ratio between 1 and 0.5. The washing step is useful to remove any salts deriving from the acidification step.

According to a preferred embodiment, the process according to the present invention includes, downstream of the isolation step (F), a dehydration step (G), which is carried out in a way chosen from: thermal evaporation, lyophilisation, or application of infrared radiation.

The cutin extract obtained from the dehydration step (G) preferably has a water content < 10%.

### Exhausted tomato peel waste, free of cutin.

A further object of the invention is the exhausted tomato peel waste according to the present invention, free of cutin, having a composition comprising or consisting of:
- water, in a concentration comprised between 75% and 85% by weight on the weight of the exhausted waste (w/w), preferably equal to about 75% (w/w);
- vegetable fibres, in a concentration comprised between 8% and 15% (w/w);
- carotenoids, in a concentration comprised between 200 mg/kg and 500 mg/kg, preferably equal to 400 mg/kg.

According to a preferred embodiment, the exhausted waste can be used as
- biomass for biogas production by anaerobic digestion, or
- biomass for the extraction of carotenoids and/or lycopene, or
- animal feed, or
- active ingredient for the production of food supplements
- additive for a variety of uses, preferably foodstuffs

Note that the cutin-free exhausted waste is that obtained from the isolation step (D) according to the process of the present invention.

### Bio-resin

A further object of the present invention is a bio-resin comprising at least the cutin extract obtained by means of the process of the present invention and an organic solvent.

Preferably, the bio-resin comprises or consists of:
*a*) a cutin extract having a 10,16-dihydroxyhexadecanoic acid purity comprised between 50% and 80% by weight on the total weight of the cutin extract (w/w); and
*b1*) a mixture of water, in concentrations comprised between 25% and 40% by volume on the total volume of the bio-resin (v/v), preferably between 20% and 35%, preferably between 25% and 35%, preferably equal to about 30%; and an organic solvent, in concentrations between 5% and 20% (v/v), preferably between 5 and 15% (v/v), preferably equal to about 10% (v/v);
or, alternatively to (*b1*),
*b2)* an organic solvent, in concentrations comprised between 5% and 20% (v/v), preferably comprised between 5% and 15% (v/v), preferably equal to about 10% (v/v).

The organic solvent is preferably chosen from the group consisting of butyl glycol, 1,3- propanediol, ethanolamine, diethylamine and ethanol. Still preferably, the organic solvent is butyl glycol or 1,3-propanediol.

### Multilayer article

A further object of the present invention is a multilayer article comprising:
- a first layer, comprising or consisting of paper, plastic, bio-plastic, metals, metal alloys, wood;
- a second layer, adjacent to the first layer, comprising the bio-resin according to the present invention.

The first layer of the multilayer article, according to the present invention, is preferably an outer layer of the article.

The second layer of the multilayer article, in accordance with the present invention, is preferably an inner layer of the article.

In particular, the inner layer is the one intended for contact with the products or foodstuffs contained therein.

The advantage of multilayer articles thus produced is that the bio-resin, comprising a cutin extract that has been obtained without the use of organic solvents or hydrochloric acid, does not cause toxicity or contamination phenomena of the food with which the bio-resin comes into contact.

The articles, in fact, are preferably food packages or wrappings.

Preferably, the multilayer article according to the present invention is chosen from the group consisting of: can, tray, bag, pouche, jar, tube, pot, cassette, bottle and drum.

### Use of the bio-resin

A further object of the present invention is the use of the bio-resin according to the present invention as:
- binder for lacquer formulations, coating and paint formulations for application on metal and wood substrates;
- binder for cosmetic formulations;
- main ingredient of water-based paints for surface treatment of plants or grass.

### EXAMPLES

For illustrative and non-limiting purposes, the results of the microbiological and chemical analysis performed on the cutin extract object of the present invention are provided.

### Microbiological analyses on cutin:

Microorganisms at 30°C (count) < 1000 UFC/gr
Salmonella spp (search) < LOQ UFC/gr
Escherichia coli beta glucuronidase positive (count) < LOQ UFC/gr
Yeasts (count) < LOQ UFC/gr

### Chemical analysis on cutin:

Sugars < 0.1 g/100 g
Total fats < 5 g/100 g
Lycopene 400-600 mg/kg

## Claims

1. Process for the extraction of cutin from tomato waste comprising the following steps:
*A.* Preparing waste or by-products obtained from tomato processing;
*B.* Immersing the tomato waste in an alkaline solution to obtain an alkaline mixture having a pH > 10, the mixture comprising a liquid phase and a dispersed solid residue;
*C*. Subjecting the alkaline mixture to a heat treatment, at a temperature > 100°C and ≤ 130°C, for a time period ≥ 10 minutes and < 4 hours;
*D.* Isolating the liquid phase of the alkaline mixture subjected to heat treatment from the solid residue, the liquid phase being a cutin alkaline solution, the solid residue being an exhausted waste of tomato peels free of cutin;
*E.* Acidifying the alkaline solution of cutin by adding an organic acid, so as to obtain an acidified suspension of cutin having a pH < 5, the acidified suspension of cutin comprising a liquid phase and a precipitate;
*F.* Isolating the precipitate of the acidified suspension of cutin from the liquid phase by decanter horizontal centrifuge and washing the precipitate with water,
the precipitate obtained being a cutin extract having a 10, 16-dihydroxyhexadecanoic acid purity between 50% and 80% by weight on the total weight of the extract (w/w).

2. Process according to claim 1, wherein the alkaline solution is an aqueous solution comprising one or more bases, selected from the group consisting of: NaOH, KOH.

3. Process according to claim 1 or 2, wherein said alkaline solution has a concentration between 0.5 and 6 M.

4. Process according to any one of claims from 1 to 3, wherein the liquid phase obtained from the isolation step (F) is
- eliminated, or
- re-entered in the immersion step (B) as an alkaline suspension of cutin, after alkalinization to pH > 10.

5. Process according to any one of claims from 1 to 4, wherein said organic acid is selected from the group consisting of: acetic acid, citric acid, formic acid, oxalic acid, succinic acid, glutaric acid, maleic acid.

6. Process according to any one of claims from 1 to 5, wherein the tomato waste or by-products are selected from the group consisting of: peels, seeds, foliage waste, green tomatoes, peels residues, and mixtures of the foregoing.

7. Process according to any one of claims from 1 to 6, comprising downstream of the isolation step (F), a dehydration step of the precipitate (G), said dehydration step (G) being conducted according to a mode selected from: thermal evaporation, lyophilisation, or application of infrared radiation.

8. Exhausted waste of tomato peels, having, when dried, a cutin content < 30% by weight on the total weight of the exhausted peel waste, and having a composition comprising or consisting of:
- water, in a concentration between 75% and 85% by weight on the weight of the exhausted waste (w/w);
- vegetable fibres, in a concentration between 8% and 15% (w/w);
- carotenoids in a concentration between 200 mg/kg and 500 mg/kg.

9. Use of the exhausted waste of tomato peels according to claim 8, as:
- biomass for the production of biogas by anaerobic digestion, or
- biomass for the extraction of carotenoids and/or lycopene, or
- animal feed, or
- active ingredient for the production of food supplements,
- food additive.

10. Bio-resin comprising or consisting of
a) a cutin extract having a 10,16-dihydroxyhexadecanoic acid purity comprised between 50% to 80% by weight on the total weight of the cutin extract (w/w), in combination with
b1) a mixture of water, in concentrations comprised between 15% and 40% by volume on the total volume of the bio-resin (v/v) and an organic solvent, in concentrations between 5% and 20% (v/v), preferably between 5% and 15% (v/v); or
b2) an organic solvent, in concentrations between 5% and 20% (v/v), preferably between 5% and 15% (v/v).

11. Multilayer article, comprising:
- a first layer comprising or consisting of paper, plastic, bioplastic, metals, metal alloys, wood;
- a second layer, adjacent to the first layer, comprising or consisting of the bio-resin according to claim 10.

## Patentansprüche

1. Verfahren zur Extraktion von Cutin aus Tomatenabfällen, umfassend die folgenden Schritte:
A. Vorbereiten von Abfällen oder Nebenprodukten aus der Tomatenverarbeitung;
B. Eintauchen der Tomatenabfälle in eine alkalische Lösung zur Herstellung eines alkalischen Gemisches mit einem pH-Wert > 10, wobei das Gemisch eine flüssige Phase und einen dispergierten Feststoffrückstand umfasst;
C. Unterziehen des alkalischen Gemisches einer Wärmeeinwirkung bei einer Temperatur > 100 °C und < 130 °C über einen Zeitraum > 10 Minuten und < 4 Stunden;
D. Abtrennen der flüssigen Phase des wärmebehandelten alkalischen Gemisches vom Feststoffrückstand, wobei die flüssige Phase eine alkalische Cutinlösung ist, der Feststoffrückstand ein erschöpfter Tomatenschalenabfall ohne Cutin ist;
E. Ansäuern der alkalischen Cutinlösung durch Zugabe einer organischen Säure, um eine angesäuerte Cutinsuspension mit einem pH-Wert < 5 zu erhalten, wobei die angesäuerte Cutinsuspension eine flüssige Phase und ein Präzipitat umfasst;
F. Abtrennen des Präzipitats der angesäuerten Cutinsuspension von der flüssigen Phase mittels horizontaler Dekanterzentrifuge und Waschen des Präzipitats mit Wasser, wobei das erhaltene Präzipitat ein Cutinextrakt mit einem Gehalt an 10,16-Dihydroxyhexadecansäure von 50 % bis 80 % des Gesamtgewichts des Extrakts (w/w) ist.

2. Verfahren nach Anspruch 1, wobei die alkalische Lösung eine wässrige Lösung ist, die eine oder mehrere Basen aus der Gruppe bestehend aus NaOH, KOH enthält.

3. Verfahren nach Anspruch 1 oder 2, wobei die alkalische Lösung eine Konzentration zwischen 0,5 und 6 M aufweist.

4. Verfahren nach einem der Ansprüche 1 bis 3, wobei die in Schritt (F) isolierte flüssige Phase
- beseitigt wird oder
- dem Eintauchschritt (B) als alkalische Cutinsuspension nach einer Alkalisierung auf pH > 10 wieder zugeführt wird.

5. Verfahren nach einem der Ansprüche 1 bis 4, wobei die organische Säure aus der Gruppe bestehend aus Essigsäure, Zitronensäure, Ameisensäure, Oxalsäure, Bernsteinsäure, Glutarsäure, Maleinsäure ausgewählt ist.

6. Verfahren nach einem der Ansprüche 1 bis 5, wobei die Tomatenabfälle oder Nebenprodukte aus der Gruppe bestehend aus Schalen, Samen, Pflanzenrückständen, grünen Tomaten, Schalenresten und deren Mischungen ausgewählt sind.

7. Verfahren nach einem der Ansprüche 1 bis 6, umfassend nach dem Isolierungsschritt (F) einen Trocknungsschritt des Präzipitats (G), wobei dieser Trocknungsschritt (G) nach einer Methode durchgeführt wird, die ausgewählt ist aus: thermischer Verdampfung, Lyophilisation oder Anwendung von Infrarotstrahlung.

8. Erschöpfte Tomatenschalenabfälle, die im getrockneten Zustand einen Cutingehalt < 30 % des Gesamtgewichts des erschöpften Schalenabfalls aufweisen und eine Zusammensetzung umfassen oder bestehen aus:
• Wasser in einer Konzentration zwischen 75 % und 85 % des Gewichts des erschöpften Abfalls (w/w);
• pflanzliche Fasern in einer Konzentration zwischen 8 % und 15 % (w/w);
• Carotinoide in einer Konzentration zwischen 200 mg/kg und 500 mg/kg.

9. Verwendung der erschöpften Tomatenschalenabfälle gemäß Anspruch 8 als:
• Biomasse zur Biogaserzeugung durch anaerobe Vergärung oder
• Biomasse zur Extraktion von Carotinoiden und/oder Lycopin oder
• Futtermittel oder
• Wirkstoff zur Herstellung von Nahrungsergänzungsmitteln,
• Lebensmittelzusatzstoff.

10. Bio-Harz, umfassend oder bestehend aus:
a) einem Cutinextrakt mit einem Gehalt an 10,16-Dihydroxyhexadecansäure zwischen 50 % und 80 % des Gesamtgewichts des Cutinextrakts (w/w), in Kombination mit
b1) einem Gemisch aus Wasser in einer Konzentration zwischen 15 % und 40 % des Gesamtvolumens des Bio-Harzes (v/v) und einem organischen Lösungsmittel in einer Konzentration zwischen 5 % und 20 % (v/v), vorzugsweise zwischen 5 % und 15 % (v/v); oder
b2) einem organischen Lösungsmittel in einer Konzentration zwischen 5 % und 20 % (v/v), vorzugsweise zwischen 5 % und 15 % (v/v).

11. Mehrschichtiger Artikel, umfassend:
- eine erste Schicht, umfassend oder bestehend aus Papier, Kunststoff, Biokunststoff, Metallen, Metalllegierungen, Holz;
- eine zweite Schicht, angrenzend an die erste Schicht, umfassend oder bestehend aus dem Bio-Harz gemäß Anspruch 10.

## Revendications

1. Procédé d'extraction de la cutine à partir de déchets de tomates, comprenant les étapes suivantes :
A. Préparer les déchets ou sous-produits issus de la transformation de la tomate ;
B. Immerger les déchets de tomates dans une solution alcaline pour obtenir un mélange alcalin ayant un pH > 10, le mélange comprenant une phase liquide et un résidu solide dispersé ;
C. Soumettre le mélange alcalin à un traitement thermique, à une température > 100°C et < 130°C, pendant une durée > 10 minutes et < 4 heures ;
D. Isoler la phase liquide du mélange alcalin ayant subi le traitement thermique du résidu solide, la phase liquide étant une solution alcaline de cutine, le résidu solide étant un déchet épuisé de pelures de tomates dépourvu de cutine ;
E. Acidifier la solution alcaline de cutine en ajoutant un acide organique, de façon à obtenir une suspension acidifiée de cutine ayant un pH < 5, ladite suspension acidifiée de cutine comprenant une phase liquide et un précipité ;
F. Isoler le précipité de la suspension acidifiée de cutine de la phase liquide à l'aide d'une centrifugeuse horizontale à décanteur et laver le précipité avec de l'eau,
le précipité obtenu étant un extrait de cutine ayant une pureté en acide 10,16-dihydroxyhexadécanoïque comprise entre 50 % et 80 % en poids par rapport au poids total de l'extrait (p/p).

2. Procédé selon la revendication 1, dans lequel la solution alcaline est une solution aqueuse comprenant une ou plusieurs bases choisies dans le groupe constitué par : NaOH, KOH.

3. Procédé selon la revendication 1 ou 2, dans lequel ladite solution alcaline a une concentration comprise entre 0,5 et 6 M.

4. Procédé selon l'une quelconque des revendications 1 à 3, dans lequel la phase liquide obtenue à l'étape d'isolement (F) est :
éliminée, ou
réutilisée à l'étape d'immersion (B) en tant que suspension alcaline de cutine, après alcalinisation à un pH > 10

5. Procédé selon l'une quelconque des revendications 1 à 4, dans lequel ledit acide or
ganique est choisi dans le groupe constitué par : acide acétique, acide citrique, acide formique, acide oxalique, acide succinique, acide glutarique, acide maléique.

6. Procédé selon l'une quelconque des revendications 1 à 5, dans lequel les déchets ou sous-produits de tomates sont choisis dans le groupe constitué par : pelures, graines, résidus végétaux, tomates vertes, résidus de pelures, et mélanges des éléments précités.

7. Procédé selon l'une quelconque des revendications 1 à 6, comprenant, en aval de l'étape d'isolement (F), une étape de déshydratation du précipité (G), ladite étape de déshydratation (G) étant réalisée selon un mode choisi parmi : évaporation thermique, lyophilisation, ou application de rayonnement infrarouge.

8. Déchets épuisés de pelures de tomates ayant, une fois séchés, une teneur en cutine < 30 % en poids par rapport au poids total des déchets de pelures épuisés, et ayant une composition comprenant ou constituée de :
• eau, à une concentration comprise entre 75 % et 85 % en poids par rapport au poids des déchets épuisés (p/p) ;
• fibres végétales, à une concentration comprise entre 8 % et 15 % (p/p) ;
• caroténoïdes à une concentration comprise entre 200 mg/kg et 500 mg/kg.

9. Utilisation des déchets épuisés de pelures de tomates selon la revendication 8, comme :
• biomasse pour la production de biogaz par digestion anaérobie, ou
• biomasse pour l'extraction de caroténoïdes et/ou de lycopène, ou
• alimentation animale, ou
• ingrédient actif pour la production de compléments alimentaires,
• additif alimentaire.

10. Bio-résine comprenant ou constituée de :
a) un extrait de cutine ayant une pureté en acide 10,16-dihydroxyhexadécanoïque comprise entre 50 % et 80 % en poids par rapport au poids total de l'extrait de cutine (p/p), en combinaison avec
b1) un mélange d'eau, à des concentrations comprises entre 15 % et 40 % en volume par rapport au volume total de la bio-résine (v/v), et un solvant organique, à des concentrations comprises entre 5 % et 20 % (v/v), de préférence entre 5 % et 15 % (v/v) ; ou
b2) un solvant organique, à des concentrations comprises entre 5 % et 20 % (v/v), de préférence entre 5 % et 15 % (v/v).

11. Article multicouche, comprenant :
- une première couche comprenant ou constituée de papier, plastique, bioplastique, métaux, alliages métalliques, bois ;
- une seconde couche, adjacente à la première, comprenant ou constituée de la bio-résine selon la revendication 10.
